# EUROPÄISCHE PATENTSCHRIFT

(11) **EP 0 376 121 B1**
(45) Veröffentlichungstag und Bekanntmachung des Hinweises auf die Patenterteilung: **09.03.1994**
(21) Anmeldenummer: 89123436.1
(22) Anmeldetag: 19.12.1989
(51) Int. Cl.: C07C 227/08, C07C 229/08

(54) **Verfahren zur Herstellung von 6-Aminocapronsäureestern**
Process for the preparation of 6-aminocaproic acid esters
Procédé pour la préparation d'esters de l'acide amino-6-capronique

(30) Priorität: 24.12.1988 DE 3843792
(43) Veröffentlichungstag der Anmeldung: 04.07.1990
(73) Patentinhaber: BASF Aktiengesellschaft, 67063 Ludwigshafen (DE)
(72) Erfinder: Merger, Franz, Dr., D-6710 Frankenthal (DE); Fischer, Rolf, Dr., D-6900 Heidelberg (DE); Harder, Wolfgang, Dr., D-6940 Weinheim (DE); Priester, Claus-Ulrich, Dr., D-6700 Ludwigshafen (DE); Vagt, Uwe, Dr., D-6720 Speyer (DE)

(56) Entgegenhaltungen:
- DE-A- 3 602 376
- DE-C- 0 952 442
- US-A- 2 777 873
- US-A- 4 766 237

## Beschreibung

Nach einem aus der US-PS 2 777 873 bekannten Verfahren erhält man 6-Aminocapronsäureester durch Umsetzen von 5-Formylvaleriansäureestern mit Ammoniak und Wasserstoff in Gegenwart von Hydrierkatalysatoren sowie Alkanolen als Lösungsmitteln unter erhöhtem Druck bei Temperaturen über 100°C. Die hierbei erzielten Ausbeuten sind noch verbesserungsbedürftig, um eine technische Realisierung zu ermöglichen. Dieses Verfahren hat weiter den Nachteil, daß die Ausbeute der aminierenden Hydrierung, die bei Einsatz von 5-Formylvalerinasäureisopropylester noch bei 70 % liegt, drastisch sinkt, wenn die Ester von 5-Formylvaleriansäure mit primären Alkanolen wie Ethanol eingesetzt werden.

Nach einem anderen in der DE-OS 36 02 378 beschriebenen Verfahren erhält man 6-Aminocapronsäureester durch Umsetzen von 5-Formylvaleriansäureestern mit Ammoniak und Wasserstoff in Gegenwart von Hydrierkatalysatoren unter Mitverwendung von Alkanolen als Lösungsmitteln bei einer Temperatur von 40 bis 95°C. Dieses Verfahren liefert zwar gute Ausbeuten, die Mitverwendung von Alkanolen hat jedoch den Nachteil, daß diese vor der Weiterverarbeitung von 6-Aminocapronsäureestern abgetrennt werden müssen und ein zusätzlicher Destillationsschritt wegen der geringen thermischen Stabilität der 6-Aminocapronsäureester unerwünscht ist. Darüber hinaus läßt die Raumzeitausbeute für eine technische Realisierung des Verfahren noch zu wünschen übrig.

Es war deshalb die technische Aufgabe gestellt, ein Verfahren zur Herstellung von 6-Aminocapronsäureestern aus 5-Formylvaleriansäureestern zur Verfügung zu stellen, bei dem hohe Raum-Zeit-Ausbeuten erzielt werden und zudem auf die Mitverwendung von Alkanolen als Lösungsmittel, die durch Destillation abgetrennt werden müssen, verzichtet werden kann.

Diese Aufgabe wird gelöst in einem Verfahren zur Herstellung von 6-Aminocapronsäureestern durch Umsetzen von 5-Formylvaleriansäureestern mit Ammoniak im überschuß und Wasserstoff in Gegenwart von Hydrierkatalysatoren bei erhöhter Temperatur und unter erhöhtem Druck, wobei man die Umsetzung in flüssigem Ammoniak als Reaktionsmedium in Gegenwart von Ruthenium-Katalysatoren durchführt.

Das neue Verfahren hat den Vorteil, daß es nicht der Mitverwendung von Alkanolen und deren Abtrennung bedarf. Weiter hat das neue Verfahren den Vorteil, daß es mit hohen Raum-Zeit-Ausbeuten verläuft, und darüber hinaus wenig Nebenprodukte gebildet werden. Ferner hat das neue Verfahren den Vorteil, daß verbesserte Katalysatorstandzeiten erzielt werden.

Bevorzugte 5-Formylvaleriansäureester sind 5-Formylvaleriansäurealkylester, insbesondere solche von C₁-C₄-Alkanolen. Geeignete Ausgangsverbindungen sind z.B. Formylvaleriansäuremethylester, -ethylester, -propylester, -isopropylester oder n-butylester. Besondere technische Bedeutung hat 5-Formylvaleriansäuremethylester erlangt.

Die Umsetzung wird in flüssigem Ammoniak durchgeführt, wobei Ammoniak sowohl als Reaktionspartner als auch als Lösungsmittel wirkt. Im allgemeinen verwendet man je kg 5-Formylvaleriansäureestern 1 bis 6 kg Ammoniak. Besonders gute Ergebnisse erhält man, wenn man je kg 5-Formylvaleriansäureester 1,2 bis 3,6, insbesondere 1,2 bis 2,4 kg Ammoniak verwendet.

Die Umsetzung wird bei erhöhter Temperatur durchgeführt. In der Regel hält man eine Temperatur von 80 bis 140°C, vorteilhaft 100 bis 135°C, insbesondere 110 bis 130°C ein.

Vorteilhaft wird je Mol 5-Formylvaleriansäureester 1 bis 20 Mol Wasserstoff angewandt. Hierbei wird die Umsetzung unter erhöhtem Druck durchgeführt, wobei vorteilhaft ein Wasserstoff-partialdruck 40 bis 1000 bar, vorzugsweise 50 bis 500 bar, insbesondere 70 bis 200 bar, eingehalten wird.

Erfindungsgemäß wird als Katalysator Ruthenium verwendet. Es ist möglich, Ruthenium in feiner Verteilung suspendiert anzuwenden. Vorzugsweise wird jedoch Ruthenium auf Trägern aufgebracht verwendet. Geeignete Träger sind beispielsweise Aluminiumoxid, Kieselgel, Titandioxid, Zirkondioxid, Magnesiumaluminate oder Magnesiumsilikate. Bevorzugt werden Aluminiumoxid und Magnesiumaluminate, insbesondere γ-Aluminiumoxid als Träger verwendet. Ruthenium wird in an sich bekannter Weise durch Tränken der Träger mit wäßrigen Lösungen von Rutheniumsalzen, wie Rutheniumchlorid oder Rutheniumnitrat, und anschließendes Trocknen und gegebenenfalls Calcinieren auf die Träger aufgebracht. Die Ruthenium-Konzentration auf dem Träger beträgt in der Regel 0,1 bis 10 Gew.-%, vorzugsweise 0,5 -5 Gew.-%, insbesondere von 1 -3 Gew.-%. Die Aktivierung der Ruthenium-Trägerkatalysatoren erfolgt in der Regel im Wasserstoffstrom zweckmäßig bei Temperaturen von 180 - 250°C, insbesondere 190 bis 230°C, z.B. innerhalb von 1 bis 20 h, vorzugsweise 1,5 bis 10 h.

In der Regel hält man eine Katalysatorbelastung von 0,1 bis 15 kg 5-Formylvaleriansäureester je kg Katalysator und Stunde ein. Besonders bewährt hat sich eine Katalysatorbelastung von 1 bis 10, insbesonders bei 4 bis 10 kg/kg.h.

Die Umsetzung kann diskontinuierlich, z. B. in einem Hochdruckgefäß durchgeführt werden. Vorzugsweise führt man die Umsetzung kontinuierlich, z.B. in einer Rührbehälterkaskade z.B. 2 bis 4 Behältern durch. Vorteilhaft hat es sich erwiesen, wenn eine Rückvermischung während der Umsetzung vermieden wird. Besonders bewährt hat es sich deshalb, wenn ein Gemisch aus 5-Formylvaleriansäureester und Ammoniak zusammen mit Wasserstoff über einen in einer rohrförmigen Reaktionszone fest angeordneten Katalysator geleitet wird. Als besonders vorteilhaft hat sich hierbei die Sumpffahrweise erwiesen. Hierbei wird in einer im wesentlichen senkrecht angeordneten rohrförmigen Reaktionszone z.B. mit einem Verhältnis von Länge : Durchmesser von 8 : 1 bis 50 : 1 in der der Katalysator fest angeordnet ist von unten 5-Formylvaleriansäureester und flüssiger Ammoniak sowie Wasserstoff zugegeben und am Kopf der rohrförmigen Reaktionszone 6-Aminocapronsäureester sowie Ammoniak und gegebenenfalls überschüssiger Wasserstoff entnommen.

Bei der bevorzugten kontinuierlichen Arbeitsweise ergibt sich die Verweilszeit aus der Katalysatorbelastung und dem Ammoniakangebot. Sie liegt vorteilhaft im Bereich von 0,5 bis 20 min, vorzugsweise von 1 bis 10, insbesondere 2 bis 6 min.

Nach Abtrennung von überschüssigem Ammonaik z.B. durch Destillation oder Strippen mit Inertgasen wie N₂ erhält man 6-Aminocapronsäureester im Gemisch mit Wasser, das bei der Reaktion anfällt.

6-Aminocapronsäureester eignen sich zur Herstellung von Caprolactam.

Das Verfahren nach der Erfindung sei an folgenden Beispielen veranschaulicht.

### Beispiel 1

Ein vertikaler Rohrreaktor Durchmesser: 16 mm, Füllhöhe: 15 cm, ölbeheizter Doppelmantel) wurde mit 10,3 g eines Katalysators aus 1,08 % Ruthenium auf Aluminiumoxid in Form von 1,5 mm Strängen gefüllt (Katalysatorherstellung : Diffusionstränkung von Aluminiumoxid mit wäßriger Rutheniumchlorid-Lösung und Trocknen bei 70°C. Der Katalysator wurde nach schrittweisem Anheben der Temperatur von 100 auf 220°C 80 min. unter Durchleiten von 50 Nl/h eines 10:1-Stickstoff-Wasserstoff-Gemisches und 120 min. unter Durchleiten von 20 Nl/h Wasserstoff bei 220°C reduziert.)
Danach wurden durch den Reaktor bei einem Druck von 98 bar und einer Temperatur von 127°C stündlich von unten nach oben unter gleichzeitigem Durchleiten von 68 Nl (3,0 mol) Wasserstoff 78,0 ml (79,9 g, 0,555 mol) 5-Formylvaleriansäuremethylester und 280 ml (168 g, 9,9 mol) flüssiges Ammoniak gepumpt.

Der Reaktionsaustrag wurde auf Normaldruck entspannt und auf den Kopf einer auf 40°C beheizten, 15 cm langen und mit 5 mm Glasringen gefüllten Kolonne geleitet, durch die im Gegenstrom 20 l/h Stickstoff geblasen wurden. Als Kolonnensumpf wurden stündlich 88,2 g wasserhaltiger Reaktionsaustrag erhalten, der nach quantitativer gaschromatographischer Analyse 80,3 % 6-Aminocapronsäuremethylester und Caprolactam enthielt, entsprechend einer Ausbeute von 88,0 % 6-Aminocapronsäuremethylester und 5,8 % Caprolactam bezogen auf eingesetzten 5-Formylvaleriansäuremethylester.

### Beispiel 2

Ein vertikaler Rohrreaktor (Durchmesser: 9 mm, Füllhöhe: 37 cm, ölbeheizter Glasmantel) wurde mit 14,8 g des in Beispiel 1 beschriebenen Katalysators mit 1,08 % Ruthenium auf Alumoniumoxid gefüllt und der Katalysator wie in Beispiel 1 beschrieben aktiviert.

Danach wurden durch den Reaktor bei einem Druck von 98 bar und einer Temperatur von 129°C stündlich von unten nach oben unter gleichzeitigem Durchleiten von 65 Nl (2,9 mol) Wasserstoff 120,0 ml (122,9 g, 0.853 mol) 5-Formylvaleriansäuremethylester und 374,6 ml (224,8 g, 13,2 mol) flüssiges Ammoniak gepumpt.

Der Reaktionsaustrag wurde auf Normaldruck entspannt und wie in Beispiel 1 beschrieben aufgearbeitet. Als Kolonnensumpf wurden stündlich 135,7 g wasserhaltiger Reaktionsaustrag enthalten, der nach quantitativer gaschromatographischer Analyse 81,0 % 6-Aminocapronsäuremethylester und 3,3 % Caprolactam enthielt, entsprechend einer Ausbeute von 88,9 % 6-Aminocapronsäuremethylester und 4,6 % Caprolactam bezogen auf eingesetzten 5-Formylvaleriansäuremethylester.

### Beispiel 3

Der in Beispiel 2 beschriebene Rohrreaktor wurde mit 17,2 g (25 ml) eines Katalysators mit 2,78 % Ruthenium auf Aluminiumoxid gefüllt, der analog Beispiel 1 hergestellt und unter Durchleiten von 20 Nl/h Wasserstoff wobei die Temperatur innerhalb von 7 h von 100 auf 220°C erhöht und dann 6 h bei 220°C aktiviert wurde.

Nach Abkühlen auf 129°C wurden durch den Reaktor bei einem Druck von 98 bar bar stündlich von unten nach oben 66 Nl (2,9 mol) Wasserstoff, 111,0 ml 5-Formylvaleriansäuremethylester (Reinheit: 98,4 %; 111,8 g, 0,776 mol) und 304,2 ml (182,5 g, 10,7 mol) flüssiges Ammoniak gepumpt.

Der Reaktionsaustrag wurde über ein Druckhalteventil auf Normaldruck entspannt und bei 20°C auf den Kopf einer 15 cm langen mit 5 mm Glasringen gefüllten Kolonne geleitet, durch die im Gegenstrom 20 l/h Stickstoff geblasen wurden. Am Kolonnensumpf wurden stündlich 125,5 g wasserhaltiger Reaktionsaustrag erhalten, der nach quantitativer gaschromatographischer Analyse 77,1 % 6-Aminocapronsäuremethylester und 3,0 % Caprolactam enthielt. Die Ausbeuten bezogen auf eingesetzten 5-Formylvaleriansäuremethylester betrug 86,0 % 6-Aminocapronsäuremethylester und 4.3 % Caprolactam.

### Beispiel 4

Der in Beispiel 2 beschriebene Rohrreaktor wurde mit 11,0 g eines Katalysators mit 1,08 % Ruthenium auf Aluminiumoxid in Form von 1,5 mm Strängen gefüllt und der Katalysator in 200 min unter schrittweisem Anheben der Temperatur von 100 auf 200°C mit 10 Nl/h Wasserstoff reduziert.

Danach wurden durch den Reaktor bei einem Druck von 98 bar und einer Temperatur von 128°C stündlich von unten nach oben unter gleichzeitigem Durchleiten von 52 Nl/h (2,3 mol) Wasserstoff 66,0 ml (67,6 g, 0,469 mol) 5-Formylvaleriansäuremethylester und 393,7 ml (236,2 g, 13,9 mol) flüssiges Ammoniak gepumpt.

Der Reaktionsaustrag wird über ein Druckhalteventil auf Normaldruck entspannt, und wie in Beispiel 1 beschrieben aufgearbeitet. Als Kolonnensumpf werden stündlich 74,6 g wasserhaltiger Reaktionsaustrag erhalten, der nach quantitativer gaschromatographischer Analyse 79,7 % 6-Aminocapronsäuremethylester und 2,6 % Caprolactam enthielt.

Die Ausbeute bez. 5-Formylvaleriansäuremethylester beträgt 87,4 % 6-Aminocapronsäuremethylester und 3,6 % Caprolactam.

### Beispiel 5

Der in Beispiel 2 beschriebene Reaktor wurde mit 17,7 g eines Katalysators mit 2,78 % Ruthenium auf Aluminiumoxid gefüllt und zur Aktivierung des Katalysators bei 40 bar unter Durchleiten von 20 Nl/h Wasserstoff die Temperatur innerhalb von 7 h von 30 auf 220°C erhöht und dann 6 h bei 220°C gehalten.

Nach Abkühlen auf 128°C wurden durch den Reaktor bei einem Druck von 98 bar stündlich von unten nach oben 53,5 Nl (2,4 mol) Wasserstoff, 108,0 ml 5-Formylvaleriansäuremethylester (Reinheit: 95 %; 105,1 g, 0,730 mol) und 240 ml (144 g, 8,5 mol) flüssiges Ammoniak gepumpt.

Der Reaktionsaustrag wurde auf Normaldruck entspannt und bei 40°C auf den Kopf einer 15 cm langen mit 5 mm Glasringen gefüllten Kolonne geleitet, durch die im Gegenstrom 20 l/h Stickstoff geblasen wurden. Am Kolonnensumpf wurden stündlich 122,1 g Reaktionsaustrag enthalten, der nach quantitativer gaschromatographischer Analyse 79,6 % 6-Aminocapronsäuremethylester und 2,8 % Caprolactam enthielt. Die Ausbeute bezogen auf eingesetzten 5-Formylvaleriansäuremethylester betrug 91,8 % 6-Aminocapronsäuremethylester und 4,2 % Caprolactam.

Das folgende Vergleichsbeispiel soll verdeutlichen, daß mit Nickel-Katalysatoren schlechtere Ergebnisse erhalten werden und darüberhinaus eine Schädigung des Hydrierkatalysators (Nickel-Austrag) eintritt. Dieses Beispiel ist nicht erfindungsgemäß:

### Vergleichsbeispiel

Ein vertikaler Rohrreaktor (Durchmesser: 16 mm, Füllhöhe: 25 cm, ölbeheizter Doppelmantel) wurde mit 54,0 g eines handelsüblichen Nickelkatalysators mit 55 Gew.-% Nickeloxid in feiner Verteilung auf Magnesiumsilikat gefüllt (in Form von Strängen von 1,5 mm Durchmesser). Der Katalysator wurde in 18 h unter schrittweisem Anheben der Temperatur von 120 auf 320°C mit 30 Nl/h eines Stickstoff-Wasserstoffgemisches (1 : 1) reduziert.

Danach wurden durch den Reaktor bei einem Druck von 98 bar und einer Temperatur von 120°C unter gleichzeitigem Durchleiten von Wasserstoff stündlich von unten nach oben 20,6 ml (21,1 g, 0,146 mol) 5-Formylvaleriansäuremethylester und 111,9 ml (67,1 g, 3,9 mol) flüssiges Ammoniak gepumpt. Das Reaktionsgemisch gelangte über einen Kühler in einen Abscheider, aus dem stündlich 11,3 l Abgas ausgeschleust wurden. Der Reaktionsaustrag wurde kontinuierlich auf den Kopf einer auf 40°C beheizten, 40 cm langen und mit 3 mm V₂A-Maschendrahtringen gefüllten Kolonne (Stripper) geleitet, durch die im Gegenstrom stündlich 20 l Stickstoff geblasen wurden. Am Kolonnensumpf wurden stündlich 23,3 g Reaktionsaustrag enthalten, der nach quantitativer gaschromatographischer Analyse 67,9 % 6-Aminocapronsäuremethylester und 6,8 % Caprolactam enthielt, entsprechend einer Ausbeute von 74,7 % 6-Aminocapronsäuremethylester und 9,6 % Caprolactam bezogen auf eingesetzten 5-Formylvaleriansäuremethylester. Der Nickel-Gehalt des Austrages beträgt 295 ppm entsprechend einem Verlust von 6,9 mg Nickel pro Stunde.

## Patentansprüche

1. Verfahren zur Herstellung von 6-Aminocapronsäureestern durch Umsetzen von 5-Formylvaleriansäurealkylestern mit Ammoniak im Überschuß und Wasserstoff in Gegenwart von Hydrierkatalysatoren bei erhöhter Temperatur unter erhöhtem Druck, dadurch gekennzeichnet, daß man die Umsetzung in flüssigem Ammoniak als Reaktionsmedium in Gegenwart von Ruthenium-Katalysatoren durchführt.

2. Verfahren nach Anspruch 1, dadurch gekennzeichnet, daß man eine Temperatur von 80 bis 140°C einhält.

3. Verfahren nach den Ansprüchen 1 und 2, dadurch gekennzeichnet, daß man einen Wasserstoffpartialdruck von 40 bis 1000 bar einhält.

4. Verfahren nach den Ansprüchen 1 bis 3, dadurch gekennzeichnet, daß man je kg 5-Formylvaleriansäureester 1 bis 6 kg flüssigen Ammoniak anwendet.

5. Verfahren nach den Ansprüchen 1 bis 4, dadurch gekennzeichnet, daß man Rutheniumträgerkatalysatoren verwendet.

6. Verfahren nach den Ansprüchen 1 bis 5, dadurch gekennzeichnet, daß man Trägerkatalysatoren mit einem Rutheniumgehalt von 0,1 bis 10 Gew.-% verwendet.

7. Verfahren nach den Ansprüchen 1 bis 6, dadurch gekennzeichnet, daß man Aluminiumoxid als Träger verwendet.

8. Verfahren nach den Ansprüchen 1 bis 7, dadurch gekennzeichnet, daß man eine Katalysatorbelastung von 0,5 bis 15 kg 5-Formylvaleriansäureester je kg Katalysator und Stunde einhält.

9. Verfahren nach den Ansprüchen 1 bis 8, dadurch gekennzeichnet, daß man 5-Formylvaleriansäurealkylester mit flüssigem Ammoniak und Wasserstoff über einen in einer rohrförmigen Reaktionszone fest angeordneten Rutheniumträgerkatalysator in Sumpffahrweise im wesentlichen unter Vermeidung von Rückvermischung leitet.

10. Verfahren nach den Ansprüchen 1 bis 9, dadurch gekennzeichnet, daß man eine Verweilzeit von 1 bis 10 min einhält.

11. Verfahren nach den Ansprüchen 1 bis 10, dadurch gekennzeichnet, daß man 5-Formylvaleriansäure C₁ bis C₄-Alkylester verwendet.

## Claims

1. A process for preparing a 6-aminocaproic ester by reacting an alkyl 5-formylvalerate with ammonia in excess and hydrogen in the presence of a hydrogenation catalyst at elevated temperature under superatmospheric pressure, which comprises performing the reaction in liquid ammonia as reaction medium and in the presence of a ruthenium catalyst.

2. A process as claimed in claim 1, wherein from 80 to 140°C is maintained.

3. A process as claimed in claim 1 or 2, wherein a hydrogen partial pressure of from 40 to 1,000 bar is maintained.

4. A process as claimed in any of claims 1 to 3, wherein from 1 to 6 kg of liquid ammonia are used per kg of 5-formylvaleric ester.

5. A process as claimed in any of claims 1 to 4, wherein a supported ruthenium catalyst is used.

6. A process as claimed in any of claims 1 to 5, wherein a supported catalyst having a ruthenium content of from 0.1 to 10% by weight is used.

7. A process as claimed in any of claims 1 to 6, wherein alumina is used as carrier.

8. A process as claimed in any of claims 1 to 7, wherein the weight hourly space velocity over the catalyst is from 0.5 to 15 kg of 5-formylvaleric ester per kg of catalyst per hour.

9. A process as claimed in any of claims 1 to 8, wherein the alkyl 5-formylvalerate is passed together with liquid ammonia and hydrogen over a fixed bed supported ruthenium catalyst in a tubular reaction zone by the liquid phase procedure with essentially no back mixing.

10. A process as claimed in any of claims 1 to 9, wherein a residence time of from 1 to 10 minutes is maintained.

11. A process as claimed in any of claims 1 to 10, wherein a C₁-C₄-alkyl 5-formylvalerate is used.

## Revendications

1. Procédé de préparation d'esters de l'acide 6-aminocaproïque par réaction d'esters alkyliques de l'acide 5-formylvalérique avec de l'ammoniac en excès et de l'hydrogène en présence de catalyseurs d'hydrogénation à température élevée sous pression élevée, caractérisé en ce qu'on effectue la réaction dans de l'ammoniac liquide comme milieu de réaction, en présence de catalyseurs au ruthénium.

2. Procédé selon la revendication 1, caractérisé en ce qu'on maintient une température de 80 à 140°C.

3. Procédé selon l'une ou l'autre des revendications 1 et 2, caractérisé en ce que l'on maintient une pression partielle d'hydrogène de 40 à 1000 bar.

4. Procédé selon l'une quelconque des revendications 1 à 3, caractérisé en ce qu'on utilise 1 à 6 kg d'ammoniac liquide par kg d'esters d'acide 5-formylvalérique.

5. Procédé selon l'une quelconque des revendications 1 à 4, caractérisé en ce qu'on utilise des catalyseurs au ruthénium supportés.

6. Procédé selon l'une quelconque des revendications 1 à 5, caractérisé en ce qu'on utilise des catalyseurs supportés ayant une teneur en ruthénium de 0,1 à 10% en poids.

7. Procédé selon l'une quelconque des revendications 1 à 6, caractérisé en ce qu'on utilise comme support de l'oxyde d'aluminium.

8. Procédé selon l'une quelconque des revendications 1 à 7, caractérisé en ce qu'on maintient une charge de catalyseur de 0,5 à 15 kg d'esters d'acide 5-formylvalérique par kg de catalyseur et par heure.

9. Procédé selon l'une quelconque des revendications 1 à 8, caractérisé en ce qu'on fait passer l'ester alkylique d'acide 5-formylvalérique avec l'ammoniac liquide et l'hydrogène sur un catalyseur supporté au ruthénium disposé à l'état solide dans une zone de réaction tubulaire, essentiellement par écoulement en évitant un mélange en retour.

10. Procédé selon l'une quelconque des revendications 1 à 9, caractérisé en ce qu'on maintient une durée de séjour de 1 à 10 minutes.

11. Procédé selon l'une quelconque des revendications 1 à 10, caractérisé en ce qu'on utilise des esters alkyliques en C₁ à C₄ de l'acide 5-formylvalérique.
